# EUROPEAN PATENT SPECIFICATION

(11) **EP 3 160 590 B1**
(45) Date of publication and mention of the grant of the patent: **25.04.2018**
(21) Application number: 15734433.4
(22) Date of filing: 29.06.2015
(51) Int. Cl.: A61P 25/28, A61P 25/16, A61K 31/194, A61K 31/205

(54) **NUTRIENTS SOLUTIONS FOR ENHANCEMENT OF COGNITIVE FUNCTION**
NÄHRSTOFFLÖSUNGEN ZUR ERHÖHUNG DER KOGNITIVEN FUNKTION
SOLUTIONS DE SUBSTANCES NUTRITIVES POUR L'AMÉLIORATION DE LA FONCTION COGNITIVE

(30) Priority: 30.06.2014 GB 201411570; 11.07.2014 GB 201412414
(43) Date of publication of application: 03.05.2017
(73) Proprietor: Mitochondrial Substrate Invention Limited, London N8 8RJ (GB)
(72) Inventor: POMYTKIN, Igor Anatolievich, London N8 8RJ (GB); CHERNOPIATKO, Anton, London N8 8RJ (GB); ANDREEVA, Larisa, London N8 8RJ (GB)
(74) Representative: Gill Jennings & Every LLP
(86) International application number: PCT/GB2015/051898
(87) International publication number: WO 2016/001643

(56) References cited:
- EP-A1- 1 321 140
- WO-A1-95/18545
- WO-A1-03/049748
- WO-A1-2010/119303
- FR-A1- 2 683 148
- US-A1- 2003 224 042
- US-A1- 2006 199 862
- US-A1- 2008 118 463
- US-A1- 2013 142 883
- DATABASE WPI Week 200621 Thomson Scientific, London, GB; AN 2006-199266 XP002743494, & JP 2006 062999 A (KOWA CO LTD) 9 March 2006 (2006-03-09)
- DATABASE WPI Week 200878 Thomson Scientific, London, GB; AN 2008-N46808 XP002743495, & RU 2 338 542 C1 (POMYTKIN I A) 20 November 2008 (2008-11-20) cited in the application
- DATABASE WPI Week 201423 Thomson Scientific, London, GB; AN 2014-E55535 XP002743496, & JP 2014 047210 A (INT SCI KK) 17 March 2014 (2014-03-17)

## Description

### Field of the Invention

The present invention relates to food industry, specifically to nutrients solutions for enhancement of cognitive function.

### Background of the invention

Cognition is a mental process related to information processing and storage, It includes knowing, specifically the process of being aware, thinking, learning, judging, ability to memory, attention, perception, action, problem solving, and mental imagery. A progressive decline of cognition is observed during normal aging. The decline in numeric ability begins at age about 25. The decline in perceptual speed begins at age about 40. The decline in inductive reasoning and spatial orientation begins at age about 50. The decline in verbal ability and verbal memory begins at age about 60. Schaie KW et al, Neuropsychol Dev Cogn B Aging Neuropsychol Cogn 2004, 11(2-3):304-24*.* Individual rates of the cognitive decline may vary. Compared to persons found to have no cognitive impairment, rate of cognitive decline during follow-up increased approximately twofold in mild cognitive impairments and fourfold in Alzheimer's disease. Wilson RS et al, Neurology 2010, 74(12):951-955*.* Early changes in cognitive functions are predictive for onset of dementia with aging, given that these changes may be observed as early as 14 years prior to diagnosis of dementia. Schaie KW et al, Neuropsychol Dev Cogn B Aging Neuropsychol Cogn 2004, 11(2-3):304-24*.* Among such early changes, diminished attention to novel events and loss of curiosity are important signs indicating the risk or onset of early stage of dementia, while increased attention to novelty is associated with successful cognitive aging and sustained interest in the novel aspects of the environment (curiosity) enhances cognitive function and diminishes cognitive decline. Nakashima Y et al, Psychogeriatrics 2010, 10:124-130*.* Daffner KR et al, J Cognitive Neuroscience 2003, 15(2):294-313*.* Daffner KR et al, Neurology 2001, 56:1377-1383*.* Daffner KR et al, J Cognitive Neuroscience 2006, 18(10):1759-1773*.* Wilson RS et al, JAMA 2002, 287(6):742-748*.* Thus, there is a great need in safe and effective agents for enhancing cognitive function to prevent or delay the onset of dementia.

Natural water is a composition of nine water isotopologues (¹H₂¹⁶O, ¹H₂¹⁷O, ¹H₂¹⁸O, ¹H¹⁶O²H, ¹H¹⁷O²H, ¹H¹⁸O²H, ²H₂¹⁶O, ²H₂¹²O , ²H₂¹⁸O) formed by stable isotopes of hydrogen ¹H and ²H (D, deuterium) and oxygen (¹⁶O, ¹⁷O, and ¹⁸O). The sum of fractional abundances of four major isotopologues ¹H₂¹⁶O (i.e. H₂O), ¹H₂¹⁸O, ¹H₂¹⁷O, and ¹H¹⁶O²H (i.e. HOD) is 0.99999952, wherein the individual abundances are as follows: 0.997317 (H₂O); 0.00199983 (¹H₂¹⁸O); 0.000372 (¹H₂¹⁷O); 0.00031069 (HOD). Rothman LS et al, J Quantitative Spectroscopy &. Radiative Transfer 2003, 82:9. Natural abundances of the rest five deuterium-bearing isotopologues (¹H¹⁷O²H, ¹H¹⁸O₂H, ²H₂¹⁶O, ²H₂¹⁷O, and ²H₂¹⁸O) are too small to be measured by currently available methods. Thus, deuterium is completely incorporated in HOD isotopologue, which content in natural water is 0.031069 mol. %.

The deuterium content in water samples is measured by isotope ratio mass-spectrometry and expressed as deuterium-to-protium ratio R=D/H, in ppm units, where D is the number of deuterium atoms, and H is the number of protium atoms. Ocean water contains deuterium at level of 155 ppm (Vienna Standard Mean Ocean Water 2, VSMOW2). Continental waters slightly differ from ocean water in deuterium content, since isotope fractionation occurs during water evaporation-precipitation process in nature. A vast majority of people reside at places, where they consume water having deuterium content from 140 to 155 ppm. Kendall et al, Hydrol Processes 2001, 15(7):1363-1393*.* Bowen et al, Water Resour Res 2007, 43*,* WO3419*.*

RU Patent No. 2338542 discloses the use of water having less than 0.018 mol.% HOD for the improvement of cognitive function and the treatment of neurodegenerative diseases. Given that deuterium in natural water is completely incorporated in HOD isotopologue, the range HOD<0.018 mol. % corresponds to the deuterium content D/H<90 ppm. JP Patent Application No. 2014047210A discloses water for injection useful for treating various diseases, including dementia, comprising deuterium-depleted water as raw material, wherein the concentration of deuterium contained in the water is 80 ppm.

Choline is an essential nutrient for healthy metabolic functioning. Choline is needed for biosynthesis of acetylcholine and essential components of cell membranes; and for methyl-group metabolism. An adequate intake level for choline is 550 mg/day for men and 425 mg/day for women. Choline deficiency leads to neurological disorders. Zeisel SH et al, Nutr Rev. 2009, 67(11): 615-623*.* US Patent Application No. 2006/0199862 A1 discloses a method for enhancing cognitive function in a mammal, comprising administering an effective amount of a choline succinate salt to the mammal. The *de novo* choline synthesis is not sufficient to meet human requirements and choline must be obtained through the diet, for example as choline salts. Choline citrate, choline bitartrate, choline succinate (2:1) salt, and choline chloride are commercially available and contain 21%, 41%, 64%, and 74% of choline, respectively. To provide fast delivery of choline to a human body, such choline supplements are frequently formulated as a beverage. However, choline salts give a specific unpleasant taste to the beverage, unacceptable to a consumer, when taken in amounts recommended by FDA (≥55 mg of choline per serving). This common disadvantage generally limits the use of choline salts as ingredients in beverages.

### Brief Description of the Drawing

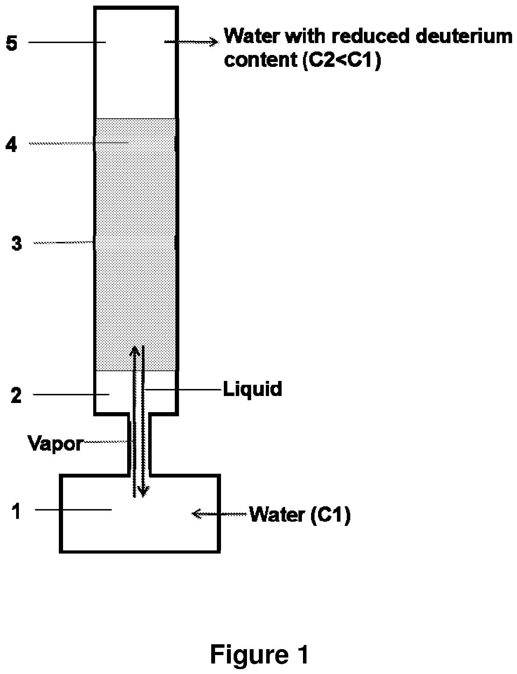

Figure 1 shows the scheme of the process for preparing water having deuterium content from 90 to about 135 ppm and up to 99.759% of isotopologue ¹H₂¹⁶O.

### Detailed Description of the Invention

The present invention provides an aqueous solution of a nutrient for use in the enhancement of cognitive function in a subject, the solution comprising a water having content of deuterium from 90 to about 135 ppm, wherein the content of ¹H₂¹⁶O isotopologue in the water is equal or less than 99.759%, and wherein the nutrient is a choline succinate salt of formula (I).

Further, the present invention provides a beverage for use in the enhancement of cognitive function in a subject, comprising (a) an aqueous solution of a nutrient and (b) water having content of deuterium from 90 to about 135 ppm and 99.759% or less of isotopologue ¹H₂¹⁶O, wherein the nutrient is a choline succinate salt of formula (I).

Further, the present invention provides a digestible capsule containing an aqueous solution of a nutrient, comprising a water having content of deuterium from 90 to about 135 ppm and 99.759% or less of isotopologue ¹H₂¹⁶O, wherein the nutrient is a choline succinate salt of formula (I).

As used herein, the term "water" refers to a composition of water isotopologues formed by stable isotopes of hydrogen (¹H and ²H) and oxygen (¹⁶O, ¹⁷O, and ¹⁸O).

As used herein, the term "isotopologue" is in accordance with IUPAC Compendium of Chemical Terminology 2nd Edition (1997) and refers to a molecular entity that differs only in isotopic composition (number of isotopic substitutions). Examples of water isotopologues include ¹H₂¹⁶O (i.e. H₂O), ¹H₂¹⁷O, ¹H₂¹⁸O, ¹H¹⁶O²H (i.e. HOD), ¹H¹⁷O²H, ¹H¹⁸O²H, ²H₂¹⁶O, ²H₂¹⁷O, and ²H₂¹⁸O.

As used herein, the term "deuterium content" refers to the amount of deuterium incorporated in water and expressed as ratio R=D/H, in ppm units, where D is the number of deuterium atoms, and H is the number of protium atoms. In practicing the invention, the deuterium content of water is from 90 to about 135 ppm, wherein term "about 135 ppm" means 135±4 ppm.

In practicing the invention, the water having deuterium content from 90 to about 135 ppm and 99.759% or less of isotopologue ¹H₂¹⁶O can be prepared by any process comprising a step of reducing the deuterium content in the natural water including, but are not limited to, a vacuum distillation of natural water.

In practicing the invention, the deuterium content can be measured by isotope ratio mass-spectrometry and expressed as ratio R=D/H, in ppm units, where D is the number of deuterium atoms, and H is the number of protium atoms.

As used herein, the term "aqueous solution" refers to any system having a water level of more than 40 wt.%, preferably more than 50 wt.%.

As used herein, the term "cognitive function" refers to any intellectual brain process involved in information processing and storage including, but are not limited to, attention, knowing, thinking, information processing, language, learning, sensing, reasoning, ability to memory, working memory, short-term memory, long-term memory, anterograde memory, retrograde memory, memory retrieval, decision-making, action, problem solving, and mental imagery.

As used herein, the term "enhancement of cognitive function" refers to enhancing any aspect of intellectual brain process involved in information processing and storage including, but are not limited to, attention, knowing, thinking, information processing, language, learning, sensing, reasoning, ability to memory, working memory, short-term memory, long-term memory, anterograde memory, retrograde memory, memory retrieval, decision-making, action, problem solving, and mental imagery.

Efficacy of an agent to enhance cognitive function can be assessed by conventional methods. In humans, the efficacy can be assessed by standardized tools such as modified mini-mental state exam, Folstein mini-mental state examination, short portable mental status questionnaire, and Alzheimer's disease assessment scale. In animals, the enhancement of cognitive function can be assessed using well-known behavior tests such as passive avoidance test, Morris water maze test, novel cage test, and modified elevated plus-maze test. Itoh J et al, Psychopharmacology 1990, 101:27-33*.* Itoh J et al, Eur J Pharmacol 1991, 194:71-76*.* Wultsch T et al, J Neural Transm. 2007, [Suppl 72]. 69-85, p.71*.* Accordingly, the effect of the aqueous solution of the invention on cognitive function can be assessed by methods well-known from the art including, but are not limited to, standardized tools such as Modified Mini-Mental State Exam, Folstein Mini-Mental State Examination, Short Portable Mental Status Questionnaire, Alzheimer's Disease Assessment Scale, Clinical Dementia Rating, Clock Drawing Test, Neuropsychiatric Inventory, Middlesex Elderly Assessment of Mental State or any similarly designed test. Instrumental measures of neuron functioning may also be used for the assessment of cognitive function including, but are not limited to, ¹H and ³¹P Nuclear Magnetic Resonance spectroscopy (NMR), Magnetic Resonance Imaging (MRI); Functional Magnetic Resonance Imaging (fMRI);Computed Tomography (CT); Computed Axial Tomography (CAT); Positron Emission Tomography (PET): Single Photon Emission Computed Tomography (SPECT); Diffuse Optical Imaging (DOI); Diffuse Optical Tomography (DOT); Z-score on the voxel-based specific regional analysis system for Alzheimer's disease (VSRAD); Exploratory eye movements recording; Event-related potentials (EPR) recording; or any similarly designed instrumentation. Using such tests, a skilled clinician would be able to assess the level of cognitive deficit of a patient or enhanced cognitive function following treatment.

In practicing the invention, water having deuterium content from 90 to about 135 ppm and isotopologue ¹H₂¹⁶O up to 99.759% may be used for enhancing cognitive function in healthy subjects and in subjects in need thereof, e.g. subjects suffering from cognitive deficits.

As used herein, the term "cognitive deficit" refers to impairment of any aspect of intellectual brain process involved in information processing and storage including, but are not limited to, attention, knowing, thinking, information processing, language, learning, sensing, reasoning, ability to memory, working memory, short-term memory, long-term memory, anterograde memory, retrograde memory, memory retrieval, decision-making, action, problem solving, and mental imagery.

Preferably, the cognitive deficit is selected from a group consisting of age-related cognitive impairment and mild cognitive impairment.

Preferably, the cognitive deficit is selected from a group consisting of Alzheimer's disease, dementia with Lewy bodies, vascular dementia, frontotemporal lobar degeneration, epilepsy, Parkinson's disease, and a prion disease.

As used herein, the term "suffering" refers to a subject who has been diagnosed with or is predisposed to a cognitive deficit. A subject may also be referred to being "at risk of suffering" from a cognitive deficit. This subject has not yet developed characteristic pathology of the cognitive deficit, however are known to be predisposing to the cognitive deficit due to family history, being genetically predispose to developing the cognitive deficit, or diagnosed with a disease or disorder that predisposes them to developing the cognitive deficit to be treated.

As used herein, the term "nutrient" refers to any compound that nourishes a living being; more specifically, nutrients are the nutritional components in foods that an organism utilizes to survive and grow. Examples of nutrients include, but are not limited to, minerals, carbohydrates, lipids, proteins, vitamins, co-factors, inorganic salts, cations and anions typically abandoned in natural drinking water, amino acids, and organic acids.

In the present invention, the nutrient is the compound of formula (I)

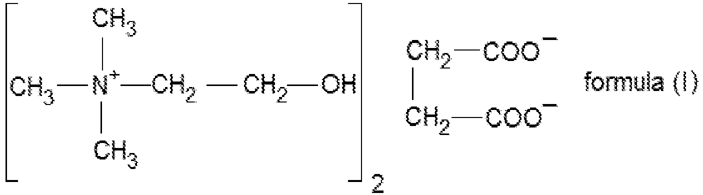

In practicing the invention, water having deuterium content within the range of 90≤D/H≤135 ppm and the content of ¹H₂¹⁶O isotopologue is equal or less than 99.759% can be used as an ingredient in synergistic combinations with the compound of formula (I) for the enhancement of cognitive function.

The compound of formula (I) can be prepared by the reaction of choline hydroxide (CAS registry No. 123-41-1) with succinic acid, as it has been described in Example 1 of the international patent application PCT/RU2007/000420, international publication number WO2009/022933A1.

In one embodiment, the aqueous solution contains from 0.01 to 50% of the compound of formula (I); preferably, from 0.1 to 50%; more preferably, from 0.1 % to 10%.

In practicing the invention, dissolution of a choline succinate salt of formula (I) in water having deuterium content from 90 to about 135 ppm and ¹H₂¹⁶O isotopologue up to 99.759% modifies the taste of this salt and makes the taste of the solution acceptable for a consumer.

In practicing the invention, the aqueous solution of a nutrient may be administered to a subject in effective amounts for a period of one day or longer.

As used herein, the term "effective amount" refers to an amount of the aqueous solution of a nutrient that, when administered to a subject will provide enhancing cognitive function. The precise effective amount will vary depending on the condition and its severity and age, weight, etc., of the subject to be treated, and the mode of administration. A physician, clinician, dietary manager, or veterinarian of ordinary skill can readily determine the effective amount of said solution for enhancing cognitive function by routine experimentation.

In practicing the invention, the aqueous solution of a nutrient may be administered to a subject in need thereof in amounts from 0.01 to 4 liters per subject per day.

In practicing the invention, the aqueous solution of the compound of formula (I) may be administered to a subject in an amount, which contains from 0.1 to 50 mg of the compound of formula (I) per kilogram of body weight of the subject.

In practicing the invention, the aqueous solution of a nutrient may be used as a component of a beverage. In one embodiment, the aqueous solution contains from 0.01 to 50% of the compound of formula (I); preferably, from 0.1 to 50%; more preferably, from 0.1 % to 10%.

As used herein, the term "beverage" refers to a substantially aqueous drinkable composition suitable for human consumption. In one embodiment, the beverage comprises at least 80% water by weight of the beverage.

In one embodiment, the aqueous solution of a nutrient is a beverage.

In practicing the invention, compound of formula (I) may be prepared as the individual substance or prepared *in situ* during a process of preparing the beverage.

When compounds of formula (I) is prepared *in situ,* an appropriate choline base or a salt thereof and an appropriate succinic acid or a salt thereof are added to the beverage to form resulting compounds (I) *in situ* in the beverage. For non-exclusive example, choline chloride and disodium succinate are added to the beverage in the proportion of 2:1 to produce the compounds (I) *in situ* in the beverage.

As used herein, the term "appropriate succinic acid salt" refers to any non-toxic succinic acid salt. Such salts include, but are not limited to, monosodium succinate, disodium succinate, potassium succinate, ammonium succinate, and mixtures and hydrates thereof.

As used herein, the term "appropriate choline salt" refers to any non-toxic choline salt. Such salts include, but are not limited to, choline hydroxide, choline chloride, choline citrate, choline bitartrate, and mixtures and hydrates thereof.

The beverage of the invention may be prepared by well-known procedures using well-known optional ingredients. Such optional ingredients generally are used individually at levels from about 0.0005% to about 10.0%, In one embodiment from about 0.005% to about 1.0% by weight of the composition. Examples of suitable optional ingredients include, but are not limited to, minerals, carbohydrates, lipids, vitamins, co-factors, buffers, flavors and sweeteners, inorganic salts, cations and anions typically abandoned in natural drinking water, taste modifying and/or masking agents, carbon dioxide, amino acids, organic acids, antioxidants, preservatives, and colorants.

Non-exclusive examples of inorganic salts typically abandoned in natural drinking water are sodium carbonate, sodium bicarbonate, potassium chloride, magnesium chloride, calcium chloride, and mixtures thereof.

Non-exclusive examples of cations are sodium, potassium, magnesium, calcium, zinc, iron, and mixtures thereof.

Non-exclusive examples of anions are fluoride, chloride, bromide, iodide, carbonate, bicarbonate, sulfate, phosphate, and mixtures thereof.

Non-exclusive examples of buffers are phosphate buffer, glycine buffer, citrate buffer, acetate buffer, carbonate buffer, tris-buffer, triethanolamine buffer, and succinate buffer.

Non-exclusive examples of flavors are synthetic flavor oils; flavoring aromatics and naturals oils such as cinnamon oil, oil of wintergreen, peppermint oils, clove oil, bay oil, anise oil, eucalyptus, thyme oil, cedar leave oil, oil of nutmeg, oil of sage, oils of citrus fruits, oil of bitter almonds, and cassia oil; plant extracts, flowers, leaves, fruits, vanilla, chocolate, mocha, coffee, apple, pear, peach, citrus such as lemon, orange, grape, lime, and grapefruit; mango, strawberry, raspberry, cherry, plum, pineapple, and apricot, and combinations thereof.

Non-exclusive examples of sweeteners are natural and synthetic sweeteners. Non-exclusive examples of natural sweeteners are naturally occurring substances, sucrose, extracts from naturally occurring substances; extracts of the plant Stevia Rebaudiana Compositae Bertoni such as stevia, steviol, rebaudiosides A-F, and dulcosides A and B; extracts of Thladiantha grosvenorii such as mogroside V and related glycosides and triterpene glycosides; phyllodulcin and its derivatives; thaumatin and its derivatives; mogrosides such as mogroside IV, mogroside V, siamenoside, and mixtures thereof; genus Siraitia including S. grosvenorii, S. siamensis, S. silomaradjae, S. sikkimensis, S. Africana, S. borneesis, and S. taiwaniana; naturally-occurring glycosides; and active compounds of plant origin having sweetening properties, and mixtures thereof. Non-exclusive examples of synthetic sweeteners are aspartame saccharin, and mixtures thereof.

Non-exclusive examples of colorants are dyes suitable for food such as those known as FD&C dyes, natural coloring agents such as grape skin extract, beet red powder, titanium dioxide, and beta-carotene, annatto, carmine, chlorophyll, paprika, and mixtures thereof.

Non-exclusive examples of organic acids are acetic acid, butyric acid, succinic acid, fumaric acid, malic acid, pyruvic acid, glutamic acid, citric acid, omega-3 unsaturated acids, linoleic acid, linolenic acid, eicosapentaenoic acid, docosahexaenoic acid, aspartic acid, and mixtures thereof.

Non-exclusive examples of amino acids are L-Tryptophan, L-Lysine, Methionine, Threonine, Levocarnitine, and L-carnitine.

Non-exclusive examples of vitamins are thiamin, riboflavin, nicotinic acid, panthothenic acid, biotin, folic acid, pyridoxine, vitamin B12, lipoic acid, vitamin A, vitamin D, vitamin E, ascorbic acid, choline, carnitine; alpha, beta, and gamma carotenes; vitamin K, and mixtures thereof.

Non-exclusive examples of co-factors are thiamine pyrophosphates, flavin mononucleotide, flavin adenine dinucleotide, nicotinamide adenine dinucleotide, pyridoxal phosphate, biotin, tetrahydrofolic acid, Coenzyme A, coenzyme B12, lipoyllysine, nicotinamide adenine dinucleotide phosphate, 11-cis-retinal, 1,25-dihydroxycholecalciferol and mixtures thereof.

Non-exclusive examples of antioxidants are Vitamin E, ascorbic acid, carotenoids, aminoindoles, Vitamin A, uric acid, flavonoids, polyphenols, herbal antioxidants, melatonin, lipoic acids, and mixtures thereof.

In practicing the invention, the aqueous solution of a nutrient may be used as a component of a dietary supplement.

As used herein, the term "dietary supplement" refers to a product taken by mouth that contains a dietary ingredient intended to supplement the diet.

In practicing the invention, the aqueous solution of a nutrient may be used as a component of a medical food. In one embodiment, the aqueous solution contains from 0.01 to 50% of the compound of formula (I); preferably, from 0.1 to 50%; more preferably, from 0.1 % to 10%.

As used herein, the term "medical food" refers to a food which is formulated to be consumed or administered enterally under the supervision of a physician and which is intended for the specific dietary management of a disease, condition, or disorder.

In practicing the invention, the aqueous solution of a nutrient may be used as a component of a pharmaceutical composition. In one embodiment, the aqueous solution contains from 0.01 to 50% of the compound of formula (I); preferably, from 0.1 to 50%; more preferably, from 0.1% to 10%.

As used herein, the term "pharmaceutical composition" refers to any composition comprising at least one pharmaceutically active ingredient and at least one other ingredient, e.g. diluent, excipient, or carrier.

In practicing the invention, water having deuterium content from 90 to about 135 ppm and ¹H₂¹⁶O isotopologue up to 99.759% is used as a component of a pharmaceutical composition at levels from 0.0001 to 99.999999% by weight of the composition.

Pharmaceutical compositions of the invention may comprise optional ingredients. Such optional ingredients generally are used individually at levels from about 0.0005% to about 10.0%, preferably from about 0.005% to about 1.0% by weight of the composition. Nonexclusive examples of optional ingredients include absorbents, buffering agents, colorants, solvents and co-solvents, coating agents, direct compression excipients, lubricants, sweetening agents, anti-fungal preservatives, antimicrobial preservatives, clarifying agents, emulsifying agents, antioxidants, surfactants, tonicity agents, and viscosity increasing agents.

In practicing the invention, the pharmaceutical composition may be prepared in a wide variety of different dosage forms including, but are not limited to, solutions, spray, aerosols, elixirs, syrups, gels, and capsules.

In practicing the invention, the pharmaceutical composition may be administered to a subject in need thereof by different routes including, but are not limited to, topical, oral, sublingual, parenteral (e.g. intravenous, subcutaneous, or intramuscular injections), nasal, rectal, vaginal, and percutaneous administration.

In practicing the invention, water having deuterium content from 90 to 135 ppm may be used in a combination with a cognitive enhancer or a mixture thereof.

As used herein, the term "cognitive enhancer" refers to any agent useful for enhancing cognitive function in a subject in need thereof.

The cognitive enhancers include, but are not limited to, agents interacting with receptors, agents interacting with enzymes, agents interacting with cytokines, agents interacting with gene expression, agents interacting with heat shock proteins, agents interacting with hormones, agents interacting with Ion channels, agents interacting with nerve growth factors, agents interacting with re-uptake transporters (psychostimulants), agents interacting with transcription factors, antioxidants, metal chelators, natural Products, nootropics ("agents without mechanism"), peptides; agents preventing amyloid-beta aggregation, e.g. ligands interacting with amyloid-beta, inhibitors of serum amyloid P component binding, vaccines against amyloid-beta, antibodies against amyloid-beta, agents interacting with tau; small molecules preventing tau aggregation, e.g. ligands interacting with tau, vaccines against tau, antibodies against tau; stem cells, and miscellaneous.

As used herein, the term "agent" refers to any substance, molecule, compound, methodology and/or biologic agent for use in the prevention, treatment, management and/ or diagnosis of a disease or disorder.

Agents interacting with receptors include, but are not limited to, agents interacting with acetylcholine receptors, e.g. muscarinic acetylcholine receptors M1, M2, M3, M4, and M5 (mAChRs) and nicotinic acetylcholine receptors alpha4beta2, alpha3beta4 and alpha7; agents interacting with adenosine receptors; agents interacting with adrenergic receptors; agents interacting with angiotensin receptors; agents interacting with cannabinoid receptors; agents interacting with chemokine receptors; agents interacting with dopamine receptors; agents interacting with endothelin receptors; agents interacting with estrogen receptors; agents interacting with GABA receptors; agents interacting with galanin receptors; agents interacting with glutamate receptors; agents interacting with glutamate receptors; agents interacting with glutamate receptors; agents interacting with G-protein coupled orphan receptors; agents interacting with histamine receptors; agents interacting with insulin receptors; agents interacting with liver X receptors; agents interacting with neurotensin receptors; agents interacting with nociceptin (ORL1) receptors; agents interacting with peripheral benzodiazepine receptors; agents interacting with peroxisome proliferator-activated (PPARs) receptors; agents interacting with prostaglandin receptors; agents interacting with purinergic receptors; agents interacting with receptor for advanced glycation end products (RAGE); agents interacting with Retinoid X receptor; agents interacting with Ryanodine receptor; agents interacting with serotonin receptors, e.g. 5-HT1A, 5-HT2, 5-HT3, 5-HT4, and 5-HT6; agents interacting with sigma receptor; agents interacting with somatostatin receptor; agents interacting with sphingosine-1-phosphate receptor; agents interacting with tachykinin receptor; agents interacting with tumor necrosis factor receptor 1; and agents interacting with any receptor to be discovered and developed to enhance cognitive function. Nonexclusive examples of agents interacting with muscarinic acetylcholine receptors M1 include orthosteric mACh M1 receptor agonists, allosteric mACh M1 receptor agonists, vincamine-type compounds, cevimeline, xanomeline, milameline, alvameline, talsaclidine, AF-267B (NGX-267), WAY-132983, sabcomeline, MCD-386, CDD-0102, azpet, AC-42, AC-260584, AC-262271, GSK-1034702, AE51090, BQCA, TBPB, AM-831, N-desmethylclozapine, vincamine, ML169, RGH-10885; and any agent to be discovered or developed in future. Nonexclusive examples of agents interacting with muscarinic acetylcholine receptors M2 include BIBN-99 and SCH-217443. Nonexclusive examples of agents interacting with nicotinic acetylcholine receptors include alpha4beta2 and alpha3beta4 nicotinic acetylcholine receptor agonists such as varenicline, pozanicline, Ispronicline, AZD-1446, Lobeline, Sofinicline, ABT-560, NS-9283, S-35836-1, SAZETIDINE-A, SR-16584, SUVN-F91201, SUVN-911. Further, nonexclusive examples of agents interacting with nicotinic acetylcholine receptors include alpha7 nicotinic acetylcholine receptor agonists such as ABT-126, EVP-6124, GTS-21, TC-5619, ABT-272, A-867744, APL-1, AZT-2, BMS-902483, BNC-1881, JNJ-1930942, PheTQS, S-24795, SEN-34625/WYE-103914, SEN-15924/WAY-361789, SKL-A4R, UCI-40083,; and any agent to be discovered or developed in future. Nonexclusive examples of agents interacting with adenosine receptors include, but are not limited to, caffeine, tozadenant, and any agent to be discovered or developed in future. Nonexclusive examples of agents interacting with adrenergic receptors include, but are not limited to, buflomedil, ORM-12741, carvedilol, and any agent to be discovered or developed in future. Nonexclusive examples of agents interacting with angiotensin receptors include, but are not limited to, tozadenant, losartan, candesartan, telmisartan, valsartan, eprosartan, irbesartan, and any agent to be discovered or developed in future. Nonexclusive examples of agents interacting with cannabinoid receptors include, but are not limited to, Dronabinol, Rimonabant, and any agent to be discovered or developed in future. Nonexclusive examples of agents interacting with chemokine receptors include, but are not limited to, RAP-310 and any agent to be discovered or developed in future. Nonexclusive examples of agents interacting with dopamine receptors include, but are not limited to, dexpramipexole, seridopidine, pridopidine, PF-03800130, and any agent to be discovered or developed in future. Nonexclusive examples of agents interacting with endothelin receptors include, but are not limited to, ENDG-6010 and any agent to be discovered or developed in future. Nonexclusive examples of agents interacting with estrogen receptors include, but are not limited to, phyto-beta-SERM, liquiritigenin, (-)-epigallocatechin-3-gallate, dehydroepiandrosterone, and any agent to be discovered or developed in future. Nonexclusive examples of agents interacting with GABA receptors include, but are not limited to, RG-1662, AC-4402, C-21191, RO4938581, UC-1011, CGP36742, CGP36742, CGP51176, CGP56433, CGP63360, (R or S)-ACBPA, HT-2157, and any agent to be discovered or developed in future. Nonexclusive examples of agents interacting with galanin receptors include, but are not limited to, HT-2157 and any agent to be discovered or developed in future. Nonexclusive examples of agents interacting with glutamate receptors include, but are not limited to, agents interacting with AMPA receptors such as Piracetam, Oxiracetam, Dimiracetam, NT-24336, Nefiracetam, AMPAkines, CX-516, CX-717, CX-1739, S-47445, CX-546, CX-554, Farampator, biarylpropylsulfonamides, PF-04958242, LY-392098, LY-404187, LY-503430, mibampator, compound 17i, compound 9a, PF-04778574, (R,R)-PIMSD, benzothiadiazides, Cyclothiazide, IDRA-21, S-18986, 1-hydroxyazoles, and any agent to be discovered or developed in future. Nonexclusive examples of agents interacting with glutamate receptors include, but are not limited to, agents interacting with NMDA receptors such as Memantine, Neramexane, EVT-103, Mnemosyne, NRX-1059, and any agent to be discovered or developed in future. Nonexclusive examples of agents interacting with glutamate receptors include, but are not limited to, agents interacting with metabotropic glutamate receptors (mGluRs) such as ADX-71149, RG-1578, RG-7090, BCI-838, BCI-632, STX-107, ADX-63365, DT-2228, RO-4491533, VU-0430644, and any agent to be discovered or developed in future. Nonexclusive examples of agents interacting with G-protein coupled orphan receptors include, but are not limited to, ESN-502, GPR3, GPR12, GPR27, GPR31, GPR52, GPR78, GPR-83, GPR135, GPR139, GPR151, GPR153, and GPR173 antagonists, Mas-related G-protein coupled receptor antagonists, RGS-14, and any agent to be discovered or developed in future. Nonexclusive examples of agents interacting with histamine receptors include, but are not limited to, ABT-288, AZD-5213, JNJ-17216498, S-38093, SAR-110894, Irdabisant, MK-3134, PD-9475, Ciproxifan, EVT-501, JNJ-10181457, SUVN-G1031, and any agent to be discovered or developed in future. Nonexclusive examples of agents interacting with insulin receptors include, but are not limited to, intranasal insulin, AGT-160, SYN-20090510RU, and any agent to be discovered or developed in future. Nonexclusive examples of agents interacting with liver X receptors include, but are not limited to, GW3965, T0901317, and any agent to be discovered or developed in future. Nonexclusive examples of agents interacting with neurotensin receptors include, but are not limited to, NT-69-L and any agent to be discovered or developed in future. Nonexclusive examples of agents interacting with nociceptin (ORL1) receptors include, but are not limited to, PF-454583, PF-4926965, and any agent to be discovered or developed in future. Nonexclusive examples of agents interacting with peripheral benzodiazepine receptors include, but are not limited to, SSR-180575, BAY-85-8102, and any agent to be discovered or developed in future. Nonexclusive examples of agents interacting with peroxisome proliferator-activated (PPARs) receptors include, but are not limited to, Rosiglitazone, Pioglitazone, Mitoglitazone, DSP-8658, G-15750, and any agent to be discovered or developed in future. Nonexclusive examples of agents interacting with prostaglandin receptors include, but are not limited to, EP2 antagonists, TG-6-10-1, and any agent to be discovered or developed in future. Nonexclusive examples of agents interacting with purinergic receptors include, but are not limited to, P2X7R antagonists, P2X7 antagonists, and any agent to be discovered or developed in future. Nonexclusive examples of agents interacting with receptor for advanced glycation end products (RAGE) include, but are not limited to, TTP-4000, DBT-066, FPS2-BM, FPS-ZM1, A-992401, humanized anti-RAGE antibody, PF-04494700, and any agent to be discovered or developed in future. Nonexclusive examples of agents interacting with Retinoid X receptor include, but are not limited to, Bexarotene, Tamibarotene, and any agent to be discovered or developed in future. Nonexclusive examples of agents interacting with Ryanodine receptor include, but are not limited to, Dantrolene and any agent to be discovered or developed in future. Nonexclusive examples of agents interacting with serotonin receptors 5-HT1A, 5-HT2, 5-HT3, 5-HT4, and 5-HT6 include, but are not limited to, F-15599, Asenapine, PRX-3140, Velusetrag, RQ-00000009, SUVN-D1003019, SUVN-1004028, Latrepirdine, Lu-AE58054, SB-742457, AVN-101, AVN-211, AVN-322, ABT-354, SAM-760, SUVN-502, SUVN-507, SYN-114, SYN-120, Pyrazolo[1,5-a]pyrimidines, 5-HT6 receptor antagonists, and any agent to be discovered or developed in future. Nonexclusive examples of agents interacting with sigma receptor include, but are not limited to, Fluvoxamine, Cutamesine, Anavex-2-73, Anavex-1-41, MC-113, (±)-PPCC, (-)-MR22, and any agent to be discovered or developed in future. Nonexclusive examples of agents interacting with somatostatin receptor include, but are not limited to, NNC-26-9100 and any agent to be discovered or developed in future. Nonexclusive examples of agents interacting with sphingosine-1-phosphate receptor include, but are not limited to, modulators of the receptor such ABT-363 and any agent to be discovered or developed in future. Nonexclusive examples of agents interacting with tachykinin receptor include, but are not limited to, SSR-241586 and any agent to be discovered or developed in future. Nonexclusive examples of agents interacting with tumor necrosis factor receptor 1 include, but are not limited to, negative allosteric modulators of the receptor such as PD-2015, PD-2016, and any agent to be discovered or developed in future.

Agents interacting with enzymes include, but are not limited to, agents interacting with Acetylcholinesterase (AChE) and Butyrylcholinesterase (BChE); agents inhibiting AChE and other biological targets; dual AChE inhibitors and AChE receptor ligands; dual AChE and amyloid-beta inhibitors; dual AChE inhibitors and antioxidants; dual AChE and beta-secretase-1 or gamma-secretase inhibitors; dual AChE inhibitors and calcium channel blockers; dual AChE inhibitors and cannabinoid receptor antagonists; dual AChE and fatty acid amide hydrolase inhibitors; dual AChE inhibitors and histamine H3 receptor antagonists; dual AChE and monoamine oxidase inhibitors; dual AChE inhibitors and metal chelators; dual AChE inhibitors and N-methyl-D-aspartic acid receptor channel blockers; dual AChE inhibitors and platelet activating factor antagonists; dual AChE and serotonin transporter inhibitors; dual AChE and sigma receptor inhibitors; agents interacting with alpha-Secretase; agents interacting with beta-Secretase; agents interacting with gamma-Secretase; gamma-Secretase inhibitors; gamma-Secretase modulators; inhibitors of gamma-Secretase activating protein; Notch pathway inhibitors; agents interacting with beta-Hexosaminidase; agents interacting with 11beta-Hydroxysteroid Dehydrogenase; agents interacting with Calpain; agents interacting with Carbonic Anhydrase; agents interacting with Caspases; agents interacting with Catechol-O-methyltransferase; agents interacting with Cathepsin; agents interacting with Cholesterol 24Shydroxylase (CYP46A1), agents interacting with Cyclooxygenase; agents interacting with D-Amino Acid Oxidase; agents interacting with Glutaminyl Cyclase; agents interacting with Glyceraldehyde-3-Phosphate Dehydrogenase; agents interacting with Glycogen Synthase Kinase-3; agents interacting with Guanylyl Cyclase; agents interacting with Heme Oxygenase; agents interacting with Histone Deacetylase; agents interacting with HMG-CoA Reductase; agents interacting with Insulin-degrading Enzyme; agents interacting with GSK-3beta; agents interacting with PKC; agents interacting with Kynurenine MonoOxygenase and Kynurenine Transaminase II; agents interacting with 5-Lipoxygenase; agents interacting with Monoamine Oxidase; agents interacting with Peptidyl-prolyl cis-trans Isomerase D; agents interacting with Phosphodiesterases; agents interacting with Phospholipases A2 and D2; agents interacting with Plasminogen Activator; agents interacting with Poly ADP-Ribose Polymerase; agents interacting with Prolyl Endo Peptidase; agents interacting with Prostaglandin D and E Synthases; agents interacting with Protein Kinase C; agents interacting with Protein Tyrosine Phosphatase; agents interacting with Rac1 GTPase; agents interacting with Ras Farnesyl Transferase; agents interacting with S-Adenosylhomocysteine Hydrolase; agents interacting with Sirtuin; agents interacting with Steroid sulfatase; agents interacting with Transglutaminase; agents interacting with Ubiquitin Carboxylterminal Hydroxylase; and agents modulating O-linked N-Acetylglucosaminidase. Nonexclusive examples of agents interacting with enzymes include Tacrine, Donepezil, Donepezil + memantine; Rivastigmine; NAL-8822; Galantamine, NAL-8801, Memogain, Huperzine A, XEL-001HP, WIN-026, Shen Er Yang, Methanesulfonyl fluoride, Bisnorcymserine, Bis-(7)-tacrine, FS-0311, Huprines, NP-0336, SPH-1285, Caproctamine, MHP-133, Ro-46-5934, NP-61, IDN-5706, IQM-622, Bisnorcymserine, Lipocrine, Memoquin, Coumarin derivatives, tacrine-chromene derivatives, ITH-4012, ITH-12118, Compound 20, MIQ-001, AMR-109, FUB833, Ladostigil, HLA20A, PMS-777, PMS-1339, RS-1259, SP-04, Etazolate, Bryostatin-1; NP-17, NP-21, NPM-01, NPM-05B1, and NPM-05B2; LY-2886721, CTS-21166, E-2609, HPP-854, MK-8931, RG-7129, AMG-0683, AZ-13, 7-aza-indole compounds; hydroxyethylamine beta-secretase-1 inhibitors; JNJ-715754; KMI-008, KMI-358, KMI-370, KMI-420, KMI-429, KMI-574, KMI-1027, and KMI-1303; L-655240, TAK-070, WAY-258131, antibodies directed against the beta-secretase cleavage site of AbetaPP; brain-targeted BACE1 antibody; Avagacestat, NIC5-15, E-2212, (-)-GSI-1; MRK-560, NGP-555, RO-02, Tarenflurbil, CHF-5074, EVP-0962, AZ-4800, BIIB-042, GSM-2, GSM-10h; SPI-014, SPI-1802, SPI-1810, and SPI-1865; IC-200155, AGT-0031, ABT-384, KR-1-2; UE-1961, UE-2811, and UE-2343; A-705253, ABT-957, AK-295, SNJ-1945, NWL-117, Tolcapone, Cerecor, Aloxistatin; Acetyl-L-leucyl-L-valyl-L-lysinal; AAV-CYP46A1, AS-2651816-00, PBD-150, PQ-912, Omigapil, Tideglusib, AX-9839; CG-9, CG-701338, CG-701446, and CG-701448; CP-70949; Dual GSK-3 /casein kinase 2 modulators; GSK-3beta inhibitors; JI-7263, NNI-362, NP-101020, SN-2127, TWS-119, VP1.15; DM-204; sGC-1016; GT-715 and GT-1061; OB-28, EVP-0334, 4-Phenylbutyrate; CHDI-390576 and CHDI-00381817; Crebinostat; KAR-3010, KAR-3084, and KAR-3166; LB-201 and LB-205; Pitavastatin, Atorvastatin, Pravastatin, Simvastatin, NST-0037, acetyl-L-carnitine, Masitinib; AIK-2, AIK-2a, AIK-2c, and AIK-21; Beta carboline alkaloids; Cadeprin, Casein kinase 1δ inhibitors; CDK5/p25 inhibitors; CZC-25146; Dasatinib; DYRK-1 alpha protein kinase inhibitors; DYRK-1 alpha protein kinase inhibitors; FRAX-120, FRAX-355, and FRAX-486; G-2019S, Hydroxy-fasudil; KIBRA pathway modulators; LDN-22684; LRRK2 inhibitors; LRRK2 inhibitors; microtubule affinity regulating kinase inhibitors; microtubule affinity regulating kinase 3 inhibitors; Minokine, NNI-351, P-005, SEL-103, SEL-141, Sorafenib, TTT-3002, URMC-099-C; protein kinase c-raf1 inhibitors; CHDI-003940246 and CHDI-00340246; PF-04859989; Minocycline, Ladostigil, RG-1577; OG-45, VAR-10200, VAR-10300, Rasagiline, Safinamide, O-GlcNAcase modulators; GlcNAcstatin, NButGT, SEG-4, Thiamet-G, Etazolate, PF-02545920, Lu-AF11167, AMG-7980, AVE-8112, BCA-909, Cilostazol, Dual PDE10/PDE 2 inhibitors; GEBR-7b, ITI-002A, IC-200214, and ITI-214; OMS-182410, PDE2A inhibitors; PDE7 inhibitors; PDE9 inhibitors; PDE10inhibitors; PF-999; Sildenafil and Tadalafil; THPP-1; Rilapladib; Icosapent ethyl; IMD-4482; E-7016; MP-124; AL-309; rosmarinic acid; HF-0220; AAD-2004; APH-0703; Bryostatin-1; DCP-LA; PKC epsilon activators; LDN-33960; Cytotoxic Necrotizing Factor 1; Sanquinarinium chloride; LNK-754; L-002259713; Resveratrol; Selisistat; INDUS-815C; DU-14; CHDI-00339864 and CHDI-00316226; Usp14 inhibitors; and any agents interacting with enzymes to be discovered and developed in future.

Agents interacting with cytokines include, but are not limited to, agents inhibiting microglia activation and production of pro-inflammatory cytokine (e.g. TNF-alpha, IL-1beta, IL-6, and IL-8). Nonexclusive examples of agents interacting with cytokines include TT-301; TT-302; Minozac; AD-16; SEN-1176; Infliximab; an antibody against the interleukin-12 subunit p40; and any agents interacting with cytokines to be discovered and developed in future.

Agents interacting with gene expression include, but are not limited to, Beperminogene perplasmid; RVX-208; AAV-CYP46A1; AZ-AAV9; HSD17B10; PRO-289; SynCav; Inventiva; and any agents interacting with gene expression to be discovered and developed in future.

Agents interacting with heat shock protein include, but are not limited to, heat shock protein 90 inhibitors PU-H71, PU-3, PU24FCl, PU-DZ8, E102, KU-32; and any agents interacting with heat shock protein to be discovered and developed in future.

Agents interacting with hormones include, but are not limited to, thyrotropin-releasing hormone and its analogues and derivatives thereof such as Taltirelin and KPS-0373; gonadotropin-releasing hormone agonist such as Leuprolide acetate implant; growth hormone releasing factor derivative such as Tesamorelin; and any agents interacting with hormones to be discovered and developed in future.

Agents interacting with ion channels (non-receptors) include, but are not limited to, blockers of L-type voltage-gated calcium channels such as ARC-029, nilvadipine, and Isradipine; ARC-031 and ARC-031-SR; RNS-60; blockers of T-type voltage-gated calcium channels such as ZSET-1446; sodium channel blockers such as AD-N02; and any agents interacting with ion channels (non-receptors) to be discovered and developed in future.

Agents interacting with nerve growth factor include, but are not limited to, NeuroAid; NeuroAiD II; CERE-110; GM-607; MIM-D3; PYM-50028; T-817MA; NsG-0202; AL-209, AL-309; MRS-001; Catecholamine derivatives; CB-1, CB-2, and CB-3; 7,8-Dihydroxy-flavone; FC29 peptide; Gambogic amine and gambogic amide; Gedunin; JRP-655; 4-methylcatechol; ND-602; and any agents interacting with nerve growth factor to be discovered and developed in future.

Agents interacting with re-uptake transporters (psychostimulants) include, but are not limited to, re-uptake inhibitors of the monoamine transporters such as Methylphenidate, Dexmethylphenidate, Modafinil, Armodafinil, Atomoxetine, Lisdexamfetamine, Indeloxazine, NS-2359, Lu-AA42202; agent interacting with creatine transporter AM-285; glycine transporter-1 inhibitors such as AS-1522489-00 and RO-4543338; dopamine transporter inhibitors such as PD-2005 and MLR-1017; selective serotonin transporter inhibitors such as Thiethylperazine, Fluoxetine, and Citalopram; and any agents interacting with re-uptake transporters (psychostimulants) to be discovered and developed in future.

Agents interacting with transcription factors include, but are not limited to, modulators of cAMP response element-binding (CREB) protein; inhibitors of exchange protein directly activated by cAMP (EPAC); and any agents interacting with transcription factor to be discovered and developed in future.

Antioxidants include, but are not limited to, acetyl-L-carnitine, curcumin, Gingko biloba extracts such as EGb 716, (R)-α-lipoic acid, melatonin, morin, trolox, vitamin C, and vitamin E, edaravone, idebenone, tirilazad, MitoQ, MitoVitE, MitoPBN, MTP-131, VP-20629; manganoporphyrine antioxidants such as AEOL-10113, AEOL-10150, AEOL-10201 and AEOL-11207; CNB-001; DL-3-n-butylphtalide; FRP-0924; IAC; Lipid soluble antioxidants; Lipocrine and Memoquin dual Acetylcholinesterase inhibitors and antioxidants; NPS-0155; PAN-811; S-52; peoniflorin; 2,2'-pyridoin; quetiapine; stemazole; zeatin; dual free radical scavengers and Abeta binding ligands; and any antioxidant to be discovered and developed in future.

Metal chelators include, but are not limited to, calcium and zinc chelators such as DP-b99, Aom-0937, PBT-2, DP-460, AEN-100; iron chelators such as Deferoxamine; non-selective metal chelators such as HLA20A, PBT-3, PBT-4; copper chelator PA-1637; dual iron-chelating agent and MAO-B inhibitors such as VAR-10200, VAR-10300; and any metal chelator to be discovered and developed in future.

Natural products include, but are not limited to, herbal extracts such as Mentat, SK-PC-B70M, KD-501, YY-280, prenylflavanone compounds PPLs, PTX-200; melatonin formulations such as melatonin and Circadin; Resveratrol; RPh-201; VR-040; Exebryl-1; Taisi; Alpha-mangostin; Anatabine; Andrographis paniculata leaves extract; Apomorphine; AX-00111; Axona; Bacopa monnieri; Baicalein; Beta-asarone; BT-11; BV-7003; Cabernet Sauvignon; Carvacrol; Catechins; Celastrol; Celastrus paniculatus; Chelerythrine; Cinnamon extract; Coumarins; Cryptotanshinone; phenolic compounds; Curcumin; DL-3-n-butylphtalide; DX-9386; Ecdysterones; (-)-Epigallocatechin-3-gallate; ESP-102; Eucommia ulmoides Oliv. Bark; Flavonoids; Fortasyn Connect; Fulvic acid; Gallic acid; Garlic extract; Gastrodin; Ginger root extracts; Gingko biloba; Grape-derived polyphenolics; Guanosine; Hederacolchidide-E; Heme; Hopeahainol A; HSH-971; HX-106; Hyperoside; IB-10C179; Icariin; IDN-5706; Kaempferol; Loganin; Luteolin; Medical Food Cocktail; Menthol; Morin; Myrcetin; Naringin; Nordihydroguaiaretic acid; 04; Obovatol; Oleuropein; Oren-gedoku-to; OroxylinA; 1,2,3,4,6-penta-O-galloyl-beta-D-glucopyranose; Piceatannol; Pinocembrin; Polyphenol derivatives; Procyanidins; Prosopis cineraria; Puerarin; Pycnogenol; Quercetin; Rosmarinic acid; Rutin; Saffron; Salidroside; S-allyl-L-cysteine; Silibinin; Sinapic acid; Souvenaid; Substance P the tachykinin undecapeptide; Syringin; Tannic acid; alpha-Tocopherol quinone; Thymol; Total coptis alkaloids; Ursolic acid; Vitamin A; Vitamin B12; Vitamin D; Withania somnifera; Wuzi Yanzong Granule; Yokukansan; Zokumei-to; bacosides A and B; Vitamin B15 (or pangamic acid), glycine, N,N-dimethyl glycine; D-cycloserine; omega-3 polyunsaturated fatty acids such as Hexadecatrienoic acid (HTA), α-Linolenic acid (ALA), Stearidonic acid (SDA), Eicosatrienoic acid (ETE), Eicosatetraenoic acid (ETA), Eicosapentaenoic acid (EPA), Heneicosapentaenoic acid (HPA), Docosapentaenoic acid (DPA), Clupanodonic acid, Docosahexaenoic acid (DHA), Tetracosapentaenoic acid, and Tetracosahexaenoic acid; and any natural product to be discovered and developed in future.

Nootrpics include, but are not limited to, LSL-001; N-251; PF-03049423; TPM-189; VI-1121; ASP-0777; RO-5508887; SEP-363856; TAK-357; AC-0523; AFX-929; AVN-457,AVN-458, andAVN-492; CPC-001; CWF-0804; D-130; D-180; GSK-2647544; J-147; JAY2-22-33; JWB1-84-1; KD-901; KU-046; LNK-3186 and LNK-3248; Maltoyl p-coumarate; MeN061016-1; MPP-26; NNZ-2591; NXD-9062; NXT-182; OG-635; Pentylenetetrazole; PNB-03, PNB-04, and PNB-05; PTI-125; RP-4000; SEL-103; SKL-A4R; TYP-1; and any nootropic to be discovered and developed in future.

Peptides include, but are not limited to, Cerebrolysin; Cortexin; Davunetide; AM-111; Etanercept; FGL; Glypromate; NNZ-2566; AL-408; Alzimag; C3bot peptides; COG-112, COG-133, and COG-1410; G-79; KIBRA pathway modulators; Leptin; MT-007; Netrin-1; NNZ-4921 and NNZ-4945; NRG-101; NT-1 and NT-2; NX-210; Pepticlere; PP-0301; RAP-310; RG-01, RG-09, and RG-018; SX-AZD1; XD4; Colostrinin; and any peptide to be discovered and developed in future.

Agents preventing Amyloid-beta aggregation include, but are not limited to, Tafamidis, Eprodisate, ARC-029, Davunetide, APH-0703, Doxycycline hyclate, ELND-005, SOM-0226, AAD-2004, Beta amyloid modulators; BLU-8499; DWP-09031; Exebryl-1; NP-61; Systebryl; Amyloid-beta oligomer cellular prion protein binding inhibitors; peptidomimetic compounds that interfere with Amyloid-beta aggregates; Amyloid-beta/tau protein aggregation inhibitors; Amyloid-derived diffusible ligands; ARN-4261 and ARN-2966; AVCRI-104P4; AVN-457,AVN-458 andAVN-492; AZP-2006; Beta-amyloid/alpha-synuclein/tau aggregation inhibitors; Beta amyloid beta sheet formation inhibitors; Beta-amyloid modulators targeting Amyloid-beta oligomers and misfolded proteins; Beta-amyloid precursor protein modulators; BMS-869780; BTA-EG4; C36; Caprospinol; Carvedilol; CLR01; CLR-097; Cotinine; Daunomycin; DBT-1339; Enoxaparin; glycosaminoglycan/carbohydrate compounds; Galantamine; haw-AD-14; HO-4160; Imipramine; IPS-04001, IPS-04001 and IPS-04003; KMS-88; Minocycline; NPT-4003; Rifampicin; Rolitetracycline; SD-1002; SEN-1500; SP-08; TAK-070; Tetracycline(s); VP-20629; AGT-160; EDN-OL1; NPT-001; compounds capable of blocking the transformation of human spherons into plaques; inhibitors of serum amyloid P component binding to amyloid fibrils such as Ro-63-8695; vaccines against amyloid-beta such as AN-1792, Affitope AD-02, CAD-106, Vanutide cridificar, ACI-24, Affitope-AD-03, UB-311, V-950, ABvac40 and ABvac42, ADepVac, ALZ-101, ALZ-301, vaccine that activates antibodies against Amyloid-beta protein, Amyloid-beta 3-10 DNA vaccination, Ankyrin G, BAN-2203, BBS-1 BACE inhibitor mAb vaccine, C12, EB-101, Glatimer acetate, MER-5101, Mimovax, NU-700, Recombinant adenovirus vector vaccine, RV-03, SeV-amyloid beta RNA vaccine; Antibodies against amyloid-beta such as Bapineuzumab, Solanezumab, Gantenerumab, Crenezumab, GSK-933776A, intravenous immunoglobulins, Immune globulin intravenous human, Octagam, Flebogamma DIF 10% (or 5%), BAN-2401, NI-101, PF-05236812, RN6G, SAR-228810, 4E10, 6F6 (GSK), 9D5, A-887755, A-992401, Ab40-4-42, ACU-193, AD-0802, AGT-160, ALZ-201, Amyloid precursor protein C-terminal fragment-targeted monoclonal antibodies, Anti-amyloid beta antibodies, Brain-targeted BACE1 antibody, CPHPC+antibody, DLX-212, Fully human monoclonal antibodies, IN-N01, IN-N01-OX2, Lpathomab, MDT-2007, naturally occurring single chain antibodies of Camelidae, NEOD-001, STC-103; and any agents preventing Amyloid-beta aggregation to be discovered and developed in future.

Ligands interacting with Tau include, but are not limited to, small molecules preventing tau aggregation such as methylene blue (TRx-0014), LMT-X, PBT-2, Tideglusib, BMS-241027, PP2A stimulators, Amyloid-beta/tau protein aggregation inhibitors, Astemizole and Lansoprazole, Berberine, Bezafibrate, BLV-0703, EpothiloneD, Fulvic acid, Insulin intranasal, L-3-n-butylphthalide, NBB BSc3504, NC-11813, NP-111001 derivatives, NPT-002, Protein phosphatase methylesterase 1 (PME1) inhibitors, Protein phosphatase 2A stimulators, tau detoxifying compounds targeting tau-mediated cytotoxicity (ReS3-T, ReS8-T, ReS10-T and ReS19-T), ReS9-S7 and ReS12-S, SIG-1012 and SIG-1106, small molecule Tau protein modulators, Sodium selenite, Tau oligomer inhibitors, Tau phosphorylation inhibitors, agents targeting tau kinase, Thiamet-G, THQ-4S and THQ-55, TRx-0237, Tubastatin A; ligands interacting with Tau such as T-777, T-807, and T-808; Vaccines against Tau such as Recombinant misfolded truncated tau protein vaccine, RV-03; Antibodies against tau and α-synuclein such as humanized tau monoclonal antibodies, NI-105, PD-0805, T01-OX2, TauC3 monoclonal antibody, Tau protein modulators, monoclonal antibody targeted to tau, TOC-1; and any ligands interacting with Tau to be discovered and developed in future.

Stem cells include, but are not limited to, GDNF/BDNF-producing glial and brain-derived stem cells, human neural stem cell, mesenchymal bone marrow-derived stem cell, NSI-189, NSI-566RSC, Neurostem-AD, adult mesenchymal precursor stem cells, allogenic umbilical cord stem cells, brain-derived stem cells, cord blood stem cells (e.g. CPG23NEUR), glial progenitor cells, human neural progenitor cells, human umbilical cord blood cells, stem cell stimulators (e.g. NBI-18), NeurotrophinCell, NGN-9079, ReN-004, ReN-005, compounds inducing stem cell differentiation (e.g. SP-sc4 and SP-sc7), Allopregnanolone, Granulocyte colony-stimulating factor, neurogenesis inductors (e.g. Valproic acid); and any stem cells to be discovered and developed in future.

Miscellaneous cognitive enhancers include, but are not limited to, autophagy inducers (e.g. JRP-900), cellular homeostasis modulators (e.g. CNS-102), glycan inhibitors, macrophage migration inhibitory factor (MIF) inhibitors (e.g. INV-88), MicroRNA (miRNA) mimetics, Proteasome-gating modulators, Synaptic vesicle glycoprotein 2A (SV2A) ligand levetiracetam, Low-dose therapy Levetiracetam, Brivaracetam; anti-inflammatory agents such as dapsone, indomethacin, nonsteroidal anti-inflammatory drugs (NSAIDs) and COX-2 inhibitors; micronutrients such as selenium and zinc; water having deuterium content from 0 to 89 ppm; water having 17-oxygen content from 0 to 500 ppm; water having 18-oxygen content from 0 to 3000 ppm; and any cognitive enhancer to be discovered and developed in future.

For enhancing cognitive function, cognitive enhancers can be used as a mixture thereof. Nonexclusive examples of such mixtures include combinations of cognitive enhancer with water selected from the group consisting of water having deuterium content from 0 to 89 ppm, water having 17-oxygen content from 0 to 500 ppm, and water having 18-oxygen content from 0 to 3000 ppm.

In practicing the invention, an effective amount of the aqueous solution of a nutrient may be encapsulated into a digestible capsule by methods well-known from the art.

In one embodiment, the aqueous solution of the compound of formula (I) may be encapsulated into a digestible capsule.

The encapsulated solution may contains the compound of formula (I) in an amount from 1 to 500 mg per capsule; preferably, from 85 to 500 mg per capsule; more preferably, from 170 to 500 mg per capsule.

As used herein, the term "encapsulation" refers to a process to entrap active agents within a carrier material to improve delivery of bioactive molecules into foods. Carrier materials used for design of protective shell of encapsulates must be food-grade, biodegradable and able to form a barrier between the internal phase and its surroundings. Nonexclusive examples of such carrier materials are polysaccharides, proteins, and lipids. The encapsulation process is well-known from the art, see e.g. Nedovich et al, Procedia Food Science 2011, 1: pages 1806-1815*.*

As used herein, the term "digestible" refers to a material that, when eaten by a subject can be broken down into compounds that can be absorbed and used as nutrients or eliminated by the subject's body. Nonlimiting examples of such digestible materials are polysaccharides, proteins, and lipids.

In one embodiment, the aqueous solution of the compound of formula (I) encapsulated into a digestible capsule is used for enhancing cognitive function in a subject.

As used herein, the term "subject" refers to any mammal including, but are not limited to, human, dog, cat, and horse. In one embodiment, the subject is a human.

The following examples are presented to demonstrate the invention. The examples are illustrative only and are not intended to limit the scope of the invention in any way.

### Example 1

This example demonstrates preparation of water having deuterium content from 90 to about 135 ppm and ¹H₂¹⁶O isotopologue up to 99.759%.

Process: the process includes steps as follows (Figure 1): evaporating the natural water with deuterium content of C1 (155 ppm) in boiling means 1 at 60°C and pressure 0.2 bars to produce water vapor; supplying the water vapor to the bottom 2 of distillation column 3; carrying out vapor-liquid contact between a descending liquid and an ascending vapor mainly on the surface of the packing 4 within the distillation column; condensing water vapor having reduced deuterium content on condenser 5 installed on upper bound of the distillation column 3; and collecting a part of condensate as condensed water having reduced deuterium content from 10 to about 70 ppm (C2), wherein C2<C1. Finally, water with deuterium content from 90 to about 135 ppm and ¹H₂¹⁶O isotopologue up to 99.759% is prepared by mixing the water having reduced deuterium content (C2) and the natural water (C1) in certain proportions.

Equipment: the distillation process, when is performed on short distillation columns 3 (e.g. 4-5 m of height), depletes natural water of deuterium-bearing isotopologues (e.g. HOD), while keeps level of H₂¹⁶O equal or less than 99.759%.

Analyses: the deuterium content in resulting water is measured by isotope mass-spectrometry and expressed as deuterium/protium ratio R=D/H, in ppm, where D is the number of deuterium atoms and H is the number of hydrogen atoms.

### Example 2

This example demonstrates a process for preparation of an aqueous solution comprising compound of formula (I) as the nutrient and water having deuterium content from 90 to about 135 ppm and ¹H₂¹⁶O isotopologue up to 99.759%.
The aqueous solution is prepared by dissolution of compound of formula (I) in water having deuterium content from 90 to about 135 ppm and ¹H₂¹⁶O isotopologue up to 99.759% (Water₉₀₋₁₃₅ₚₚₘ) in proportions as indicated in Table 1.

**Table 1**

| Ingredient | Content, wt. % |
|---|---|
| Compound of formula (I) | 0.11 |
| Water₉₀₋₁₃₅ₚₚₘ | 99.89 |

### Example 3

This example demonstrates a process for preparation of beverage comprising compound of formula (I) as the nutrient and water having deuterium content from 90 to about 135 ppm and ¹H₂¹⁶O isotopologue up to 99.759%.
The beverage is prepared by dissolution of compound of formula (I) in water having deuterium content from 90 to about 135 ppm and ¹H₂¹⁶O isotopologue up to 99.759% (Water₉₀₋₁₃₅ₚₚₘ) in proportions as indicated in Table 2. The resulted product is bottled in bottles of 330 ml volume.

**Table 2**

| Ingredient | Content, wt. % |
|---|---|
| Compound of formula (I) | 0.1 |
| Water₉₀₋₁₃₅ₚₚₘ | 99.9 |

This beverage, when is administered orally to a subject for enhancement of cognitive functions in daily amount of 330 ml per serving, provides about 210 mg of choline as the essential nutrient and can be used as a dietary supplement or a medical food for dietary management of cognitive function.

### Example 4

This example demonstrates that beverage, which comprises compound of formula (I) as the nutrient and water having deuterium content from 90 to about 135 ppm and ¹H₂¹⁶O isotopologue up to 99.759, has an improved taste.
The comparison of tastes of beverages, which were prepared on waters having different deuterium contents, but contained the same amounts of compound of formula (I), was made in blinded experiment. Test beverage was prepared as 0.1% solution of compound of formula (II) in water having deuterium content of 91 ppm and ¹H₂¹⁶O isotopologue up to 99.759% and poured in 7 cups ("Beverage D/H 91 ppm"). Control beverage was prepared as 0.1% solution of compound of formula (II) in water having deuterium content of 150 ppm and ¹H₂¹⁶O isotopologue up to 99.759% and poured in 7 cups ("Beverage D/H 150 ppm, control"). All 14 cups were randomly labeled by the numbers 1 through 14. Another cup was filled with control beverage ("Beverage D/H 150 ppm") and labeled as "the reference". During the test procedure, the consumer performs comparison of taste of the reference beverage with taste of samples from cups labeled by numbers from 1 through 14 and rates the difference in taste on scale of -1 (much more unpleasant than the reference) to +1 (much less unpleasant than the reference). A score of 0 indicates the taste of sample is equally as unpleasant as the reference. Results were treated by Mann-Whitney non-parametric analyses. Data are presented in Table 3 as mean±SEM of taste scores.

**Table 3**

| Beverage | Taste scores |
|---|---|
| Beverage D/H 150 ppm (control) | 0.14 ± 0.14 |
| Beverage D/H 91 ppm | 0.72 ± 0.18* |

| | |
|---|---|
| *Differs significantly of control (P<0.05) | |

Table 3 shows that taste of the beverage is significantly much less unpleasant, when this beverage is prepared on water having reduced deuterium content, as compared to the taste of control beverage. Thus, the beverage containing choline succinate salts in amounts effective for ameliorating choline deficiency has the taste acceptable for a consumer, when this beverage is prepared on water from 90 to about 135 ppm and ¹H₂¹⁶O isotopologue up to 99.759.

### Example 5

This example demonstrates a digestible capsule filled with the compound of formula (I) as the nutrient and water having deuterium content from 90 to about 135 ppm and ¹H₂¹⁶O isotopologue up to 99.759%. Digestible capsules were filled with 50% aqueous solution of the compound of formula (I) in water having deuterium content from 90 to about 135 ppm and ¹H₂¹⁶O isotopologue up to 99.759% (Water₉₀₋₁₃₅ₚₚₘ) in amounts of from 2 to 1000 mg per capsule.

### Example 6 (comparative example)

This example demonstrates that water having deuterium content from 90 to about 135 ppm and ¹H₂¹⁶O isotopologue less than 99.759% is useful for enhancing cognitive function in adult healthy subjects.
Young adult male Wistar rats were treated with water having D/H ratio 140.7 ppm (control group, n=10) or low deuterium water with D/H ratio 90.2 ppm (experimental group, n=10) *per os* as drinking water, 9 days treatment period, *ad libitum.* Both waters have level of ¹H₂¹⁶O isotopologue less than 99.759%. Control and experimental groups were tested for spatial learning and memory in the modified elevated plus-maze test at day 8^{th} and 9^{th} (acquisition and retention session, respectively), as it has been described by Itoh et al. Itoh et al, Psychopharmacology 1990, 101:27-33*;* Itoh et al, Eur J Pharmacol 1991, 194:71-76*.* The time for the rat to move from the open arm to the enclosed arm (transfer latency) was used as a parameter of retention and consolidation of memory. Behavioral data were treated by Mann-Whitney non-parametric analyses. Both control and experimental group exhibited significantly decreased transfer latencies on the retention session (TL2) (p<0.05), as compared to the acquisition session (TL1), meaning that rats of both groups remembered the configuration of the open and enclosed arms. However, rats of experimental group exhibited significantly shortened transfer latency on the retention session (TL2), as compared to the control group (p=0.02; Z=2.31). Data are presented in Table 4 as mean±SEM of transfer latencies on days of acquisition session (TL1) and retention session (TL2).

**Table 4**

| Group | Transfer latency, s | |
|---|---|---|
| | TL1 | TL2 |
| D/H = 140.7 ppm (control) | 62.0 ± 5.4 | 28.5 ± 5.8 |
| D/H = 90.2 ppm | 55.6 ± 6.8 | 13.5 ± 2.1 * |

| | | |
|---|---|---|
| *Differs significantly of control TL2 (p<0.05). | | |

Table 4 shows that administering water having deuterium content from 90 to about 135 ppm and ¹H₂¹⁶O isotopologue less than 99.759% is useful for enhancing cognitive function in healthy subjects.

### Example 7

### (comparative example)

This example demonstrates that water having deuterium content from 90 to about 135 ppm and ¹H₂¹⁶O isotopologue less than 99.759% is useful for enhancing cognitive function in adult healthy subjects.
Three-months-old male C57BI6J mice were treated with water having D/H ratio 153.0 ppm (control group, n=15) or low deuterium waters with D/H ratio 91.7 ppm, 119.9 ppm, and 134.9 ppm (experimental groups, n=15 per group) *per os* as drinking water, 14 days treatment period, *ad libitum.* All waters have level of ¹H₂¹⁶O isotopologue less than 99.759%. Control and experimental groups were tested on attention to novel events in the novel cage test. Mice were introduced into a standard plastic cage the size of their home cage filled with small amounts of fresh sawdust. The testing was carried out in a dark quiet room in morning hours. Behavior was videotaped and analyzed by trained observers blind to the treatment protocol. The number of exploratory rearings counted under red light during a 5-min period was used as measure of attention to novel events. Behavioral data were treated by Mann-Whitney non-parametric analyses. Data are presented in Table 5 as mean±SEM of number of exploratory rearings.

**Table 5**

| Group | Number of exploratory rearings |
|---|---|
| D/H = 153.0 ppm (control) | 30,6 ± 1,1 |
| D/H = 134.9 ppm | 33,4 ± 1,6* |
| D/H = 119.9 ppm | 35,4 ± 1,6* |
| D/H = 91.7 ppm | 35,1 ± 1,5* |

| | |
|---|---|
| *Differs significantly of control (p<0.05). | |

Table 5 shows that water having deuterium content from 90 to about 135 ppm and ¹H₂¹⁶O isotopologue less than 99.759% significantly enhances cognitive function, as compared to the control treatment.

### Example 8 (comparative example)

This example demonstrates that water having deuterium content from 90 to about 135 ppm and ¹H₂¹⁶O isotopologue less than 99.759% is useful for enhancing cognitive function in aged healthy subjects. Eighteen-months-old male C57BI6J mice were treated with water having D/H ratio 140.7 ppm (control group, n=15) or low deuterium water with D/H ratio 91.6 ppm (experimental group, n=15) *per os* as drinking water, 14 days treatment period, *ad libitum.* Both waters have level of ¹H₂¹⁶O isotopologue less than 99.759%. Control and experimental groups were tested for attention to novel events as indicated in the Example 6. Data are presented in Table 6 as mean±SEM of number of exploratory rearings for the first minute of the observations.

**Table 6**

| Group | Number of exploratory rearings |
|---|---|
| D/H = 140.7 ppm (control) | 5,91 ± 0,59 |
| D/H = 91.6 ppm | 8,45 ± 0,83* |

| | |
|---|---|
| *Differs significantly of control (p<0.05). | |

Table 6 shows that water having deuterium content from 90 to about 135 ppm and ¹H₂¹⁶O isotopologue less than 99.759% significantly enhances cognitive function in healthy aged subjects, as compared to the control treatment.

### Example 9

This example demonstrates that aqueous solution containing compound of formula (I) as the nutrient and water having deuterium content from 90 to about 135 ppm and ¹H₂¹⁶O isotopologue less than 99.759% is synergistically useful for enhancing cognitive function. Cognitively healthy young adult male Wistar rats weighing 260-280 g were randomly ascribed to groups and supplemented daily with aqueous solution of 5 mg/kg of compound of formula (I) in water of the invention for seven consecutive days. Then, after the break in the supplementation for next seven days, scopolamine 1 mg/kg i.p. or vehicle was administered (at day 14) 30 min before the acquisition trial in the passive avoidance test ("Compound (I) + Scopolamine" group). In "Vehicle + Vehicle" group, rats received vehicle for seven consecutive days and then vehicle 30 min before the acquisition trial in the passive avoidance test at 14^{th} day. In "Vehicle + Scopolamine" group, rats received vehicle for seven consecutive days and then scopolamine 1 mg/kg i.p. 30 min before the acquisition trial in the passive avoidance test at 14th day. Scopolamine is anticholinergic agent that is used commonly to produce acute disturbance of cholinergic neurotransmission, particularly the processes of learning acquisition and short-term memory (Alzheimer's disease-like state). Passive avoidance test requires the rats to behave contrary to their innate tendencies for preference of dark areas and avoidance of bright ones. The apparatus used in this test is composed by a dark compartment and a bright compartment. The latency to enter the dark chamber was measured using 300 s maximum trial duration. In the acquisition trial, the animal received a 0.4 mA electric shock for 3 s when entering the dark chamber. Retention trial was performed 24 h after the acquisition trial. Memory performance is positively correlated with the latency to escape from the bright compartment during retention trials; the greater the latency, the better the recollection. There were no differences between groups in latency to enter the dark chamber during the acquisition trial. However, there were differences in latency to enter the dark chamber during the retention trial, indicating that scopolamine induced disturbance of memory. Data are presented in Table 7 as individual latencies, range, and mean ± SEM to enter to the dark compartment during the retention trial.

**Table 7**

| | Groups | | |
|---|---|---|---|
| | Vehicle + Vehicle | Vehicle + Scopolamin e | Aqueous solution of Compound(l) + Scopolamine |
| Individual latencies, s | 300 | 300 | 55.2 |
| | 300 | 76 | 282.7 |
| | 300 | 99.8 | 295.5 |
| | 300 | 72.7 | 269.8 |
| | 300 | 300 | 300 |
| | 300 | 186.3 | 300 |
| | 207.3 | 171.4 | 196.8 |
| | 300 | 300 | 300 |
| | 226.7 | 29.1 | 41.4 |
| | 300 | 79.5 | 66 |
| | 300 | | 300 |
| Latencies range | 207.3 - 300 | 29.1 - 300 | 41.4 - 300 |
| Latency mean ± SEM | 284.9 ± 10.2 | 161.5 ± 33.6 | 218.9 ± 33.2 |

Latencies to enter the dark chamber in the retention trial in cognitively healthy rats from group "Vehicle + Vehicle" were accepted as normal ones (207.3 - 300 s), while a latency less than 207 s, i.e. less than minimal one observed in the cognitively healthy rats, was accepted as disturbance of cognitive function. Rates of disturbance of cognitive function (latency <207 s) were found to be 0.70 and 0.36 for "Vehicle+Scopolamine" and "Compound (I) + Scopolamine" groups, respectively. Relative risk of disturbance of cognitive function in "Compound (I) + Scopolamine" group as compared to "Vehicle + Scopolamine" group was found to be 0.51. Note, the 7-days-break between the last day of the supplementation and the day of onset of scopolamine-induced disturbance of cognitive function completely excludes any effect of the compound of formula (I) on animals in the diseased state, given that the elimination half-life for the compound of formula (I) is less than 1.2 h. Kovalev et al, Exp Clin Pharm 2014, 77(11):23*.* Thus, administering the aqueous solution of the compound of formula (I) as the nutrient resulted in almost 2-fold reducing the risk of disturbance of cognitive function as compared to the control.

### Example 10 (comparative example)

This example demonstrates the use of water with deuterium content from 90 to about 135 ppm and ¹H₂¹⁶O isotopologue less than 99.759% for preparing the dietary supplement for enhancing cognitive function. The dietary supplement is prepared by dissolution of calcium chloride, magnesium chloride, and sodium bicarbonate in water having deuterium content within the range of 90-135 ppm and ¹H₂¹⁶O isotopologue less than 99.759% (Water₉₀₋₁₃₅ₚₚₘ) in proportions as indicated in Table 8. The resulted product is bottled in bottles of 330 ml volume.

**Table 8**

| Ingredient | Content, wt. % |
|---|---|
| Water₉₀₋₁₃₅ₚₚₘ | 99.953 |
| Calcium Chloride | 0.015 |
| Magnesium Chloride | 0.007 |
| Sodium Bicarbonate | 0.025 |

### Example 11 (comparative example)

This example demonstrates the use of water with deuterium content from 90 to about 135 ppm and ¹H₂¹⁶O isotopologue less than 99.759% for preparing medical food for enhancing cognitive function.
The medical food is prepared by dissolution of calcium chloride, magnesium chloride, and sodium bicarbonate in water having deuterium content within the range of 90-135 ppm and ¹H₂¹⁶O isotopologue less than 99.759% (Water₉₀₋₁₃₅ₚₚₘ) in proportions as indicated in Table 9. The resulted product is bottled in bottles of 330 ml volume.

**Table 9**

| Ingredient | Content, wt. % |
|---|---|
| Water₉₀₋₁₃₅ₚₚₘ | 99.953 |
| Calcium Chloride | 0.015 |
| Magnesium Chloride | 0.007 |
| Sodium Bicarbonate | 0.025 |

The medical food is administered orally to a subject at a high risk of dementia in daily amounts from 0.01 to 4 liters for enhancing cognitive function.

### Example 12 (comparative example)

This example demonstrates the use of water with deuterium content from 90 to about 135 ppm and ¹H₂¹⁶O isotopologue less than 99.759% for preparing the pharmaceutical composition for enhancing cognitive function. The pharmaceutical composition for enhancing cognitive function is prepared by dissolution of insulin in water having deuterium content within the range of 90-135 ppm and ¹H₂¹⁶O isotopologue less than 99.759% (Water₉₀₋₁₃₅ₚₚₘ) in proportions as indicated in Table 10 for unit dosage form.

**Table 10**

| Ingredient | Content |
|---|---|
| Insulin | 20 IU |
| Water₉₀₋₁₃₅ₚₚₘ | 200 µL |

The composition is administered intranasally to a subject in need thereof once-a-day for enhancing cognitive function.

## Claims

1. An aqueous solution of a nutrient for use in the enhancement of cognitive function in a subject, the solution comprising a water having content of deuterium from 90 to 135 ppm, wherein the content of ¹H₂¹⁶O isotopologue in the water is equal or less than 99.759%, and
wherein the nutrient is the compound of formula (I)

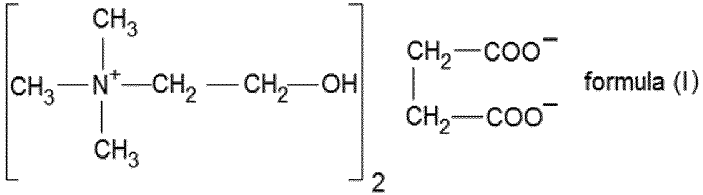

2. The aqueous solution, for use according to claim 1, wherein the subject is a healthy subject or subject suffering from a cognitive deficit.

3. The aqueous solution, for use according to claim 1, wherein the subject is suffering from a cognitive deficit selected from the group consisting of age-related cognitive impairment, mild cognitive impairment; dementia such as Alzheimer's disease, dementia with Lewy bodies, vascular dementia, frontotemporal lobar degeneration; epilepsy, Parkinson's disease, and a prion disease.

4. The aqueous solution, for use according to any of claims 1-3, wherein the solution contains from 0.01 to 50% of the compound of formula (I).

5. The aqueous solution, for use according to any of the preceding claims, which is a component of a dietary supplement, component of a medical food or beverage, or component of a pharmaceutical composition.

6. The aqueous solution, for use according to any of the preceding claims, wherein said aqueous solution is a beverage.

7. The aqueous solution, for use according to any of claims 1- 5, wherein the solution is encapsulated in a digestible material.

8. The aqueous solution, for use according to claim 7, wherein the encapsulated solution contains the compound of formula (I) in an amount from 1 to 500 mg per capsule.

9. A digestible capsule containing an aqueous solution of a nutrient, comprising a water having content of deuterium from 90 to 135 ppm and 99.759% or less of isotopologue ¹H₂¹⁶O, wherein the nutrient is the compound of formula (I)

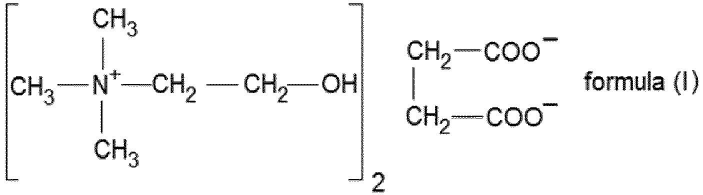

10. The digestible capsule according to claim 9, wherein the solution contains the compound of formula (I) in an amount from 1 to 500 mg per capsule.

11. The digestible capsule according to any of claims 9 or 10, for use in the enhancement of cognitive function in a subject.

12. An aqueous solution of a nutrient, wherein the nutrient is the compound of formula (I) 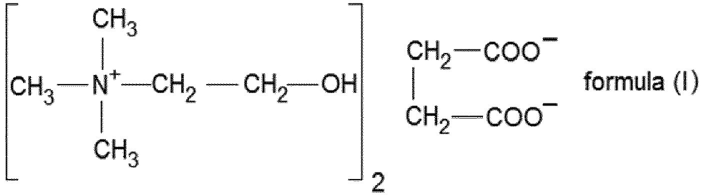 and the solution comprises a water having content of deuterium from 90 to 135 ppm and ¹H₂¹⁶O isotopologue up to 99.759%.

## Patentansprüche

1. Wasserhaltige Lösung eines Nährstoffs zur Verwendung beim Verbessern einer kognitiven Funktion in einem Subjekt, wobei die Lösung ein Wasser umfasst, das einen Gehalt von Deuterium von 90 bis 135 ppm aufweist, wobei der Gehalt von ¹H₂¹⁶O-Isotopolog in dem Wasser höchstens 99,759 % ist, und
wobei der Nährstoff die Verbindung der Formel (I) ist

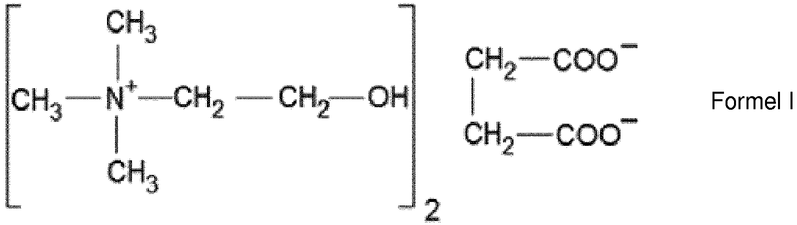

2. Wasserhaltige Lösung zur Verwendung nach Anspruch 1, wobei das Subjekt ein gesundes Subjekt ist oder ein Subjekt ist, das an einem kognitiven Defizit leidet.

3. Wasserhaltige Lösung zur Verwendung nach Anspruch 1, wobei das Subjekt an einem kognitiven Defizit leidet, ausgewählt aus der Gruppe bestehend aus altersbedingter kognitiver Beeinträchtigung, schwacher kognitiver Beeinträchtigung; Demenz, wie etwa Alzheimer-Krankheit, Lewy-Körper-Demenz, vaskulärer Demenz, frontotemporaler lobärer Degeneration; Epilepsie, Parkinson-Syndrom und einer Prionenkrankheit.

4. Wasserhaltige Lösung zur Verwendung nach einem der Ansprüche 1-3, wobei die Lösung 0,01 bis 50 % der Verbindung von Formel (I) enthält.

5. Wasserhaltige Lösung zur Verwendung nach einem der vorhergehenden Ansprüche, die ein Bestandteil eines Nahrungsergänzungsmittels, ein Bestandteil einer medizinischen Ernährung oder eines medizinischen Getränks oder ein Bestandteil einer pharmazeutischen Zusammensetzung ist.

6. Wasserhaltige Lösung zur Verwendung nach einem der vorhergehenden Ansprüche, wobei die wasserhaltige Lösung ein Getränk ist.

7. Wasserhaltige Lösung zur Verwendung nach einem der Ansprüche 1-5, wobei die Lösung in einem verdaulichen Material eingekapselt ist.

8. Wasserhaltige Lösung zur Verwendung nach Anspruch 7, wobei die eingekapselte Lösung die Verbindung von Formel (I) in einer Menge von 1 bis 500 mg pro Kapsel enthält.

9. Verdauliche Kapsel, eine wasserhaltige Lösung eines Nährstoffs enthaltend, die ein Wasser mit einem Gehalt von Deuterium von 90 bis 135 ppm und 99,759 % oder weniger von Isotopolog ¹H₂¹⁶O umfasst, wobei der Nährstoff die Verbindung von Formel (I) ist:

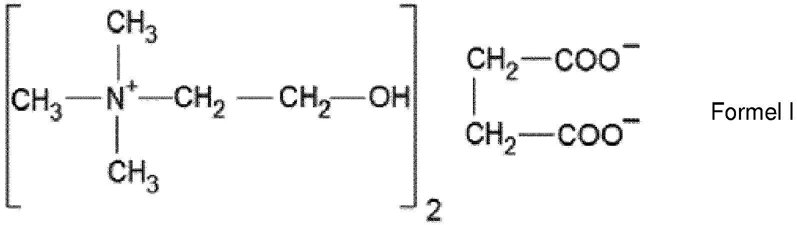

10. Verdauliche Kapsel nach Anspruch 9, wobei die Lösung die Verbindung von Formel (I) in einer Menge von 1 bis 500 mg pro Kapsel enthält.

11. Verdauliche Kapsel nach einem der Ansprüche 9 und 10 zur Verwendung beim Verbessern einer kognitiven Funktion in einem Subjekt.

12. Wasserhaltige Lösung eines Nährstoffs, wobei der Nährstoff die Verbindung von Formel (I) ist: 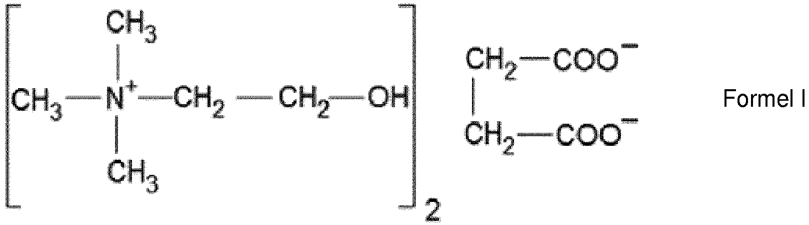 und die Lösung ein Wasser mit einem Gehalt von Deuterium von 90 bis 135 ppm und ¹H₂¹⁶O-Isotopolog bis zu 99,759 % umfasst.

## Revendications

1. Solution aqueuse d'une substance nutritive à utiliser dans l'amélioration de la fonction cognitive chez un sujet, la solution comprenant de l'eau ayant une teneur en deutérium comprise entre 90 et 135 ppm, dans laquelle la teneur en isotopologue de ¹H₂¹⁶O dans l'eau est inférieure ou égale à 99,759 %, et
dans laquelle la substance nutritive est le composé de la formule (I)

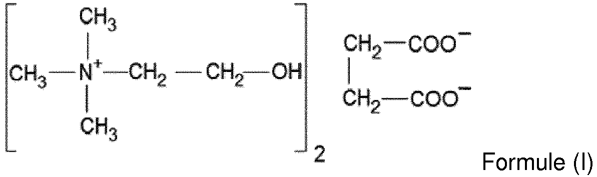

2. Solution aqueuse, à utiliser selon la revendication 1, dans laquelle le sujet est un sujet sain ou un sujet souffrant d'un déficit cognitif.

3. Solution aqueuse, à utiliser selon la revendication 1, dans laquelle le sujet souffre d'un déficit cognitif choisi dans le groupe constitué du trouble cognitif lié à l'âge, du trouble cognitif léger ; de la démence telle que la maladie d'Alzheimer, de la démence à corps de Lewy, de la démence vasculaire, de la dégénérescence lobaire fronto-temporale ; de l'épilepsie, de la maladie de Parkinson et de la maladie à prion.

4. Solution aqueuse, à utiliser selon l'une quelconque des revendications 1 à 3, dans laquelle la solution contient de 0,01 à 50 % du composé de la formule (I).

5. Solution aqueuse, à utiliser selon l'une quelconque des revendications précédentes, qui est un composant d'un complément alimentaire, un composant d'un aliment ou d'une boisson thérapeutique ou un composant d'une composition pharmaceutique.

6. Solution aqueuse, à utiliser selon l'une quelconque des revendications précédentes, ladite solution aqueuse étant une boisson.

7. Solution aqueuse, à utiliser selon l'une quelconque des revendications 1 à 5, la solution étant encapsulée dans une matière digestible.

8. Solution aqueuse, à utiliser selon la revendication 7, dans laquelle la solution encapsulée contient le composé de la formule (I) dans une quantité comprise entre 1 et 500 mg par capsule.

9. Capsule digestible contenant une solution aqueuse d'une substance nutritive, comprenant de l'eau ayant une teneur en deutérium comprise entre 90 et 135 ppm et une teneur en isotopologue de ¹H₂¹⁶O inférieure ou égale à 99,759 %, la substance nutritive étant le composé de la formule (I)

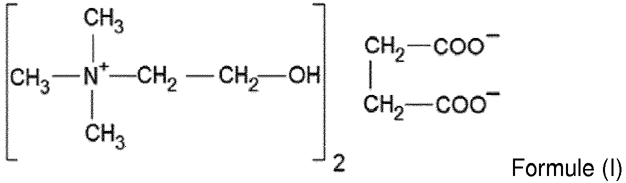

10. Capsule digestible selon la revendication 9, dans laquelle la solution contient le composé de la formule (I) dans une quantité comprise entre 1 et 500 mg par capsule.

11. Capsule digestible selon l'une quelconque des revendications 9 et 10, à utiliser dans l'amélioration de la fonction cognitive chez un sujet.

12. Solution aqueuse d'une substance nutritive, la substance nutritive étant le composé de la formule (I) 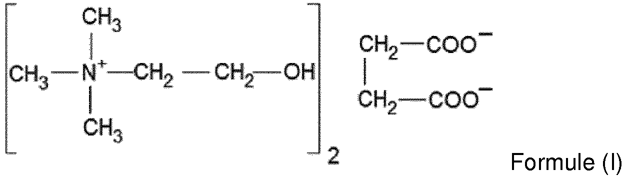 et la solution comprend de l'eau ayant une teneur en deutérium comprise entre 90 et 135 ppm et une teneur en isotopologue de ¹H₂¹⁶O de 99,759 % maximum.
